# EUROPEAN PATENT APPLICATION

(11) **EP 2 174 664 A1**
(43) Date of publication of application: **14.04.2010**
(21) Application number: 08166041.7
(22) Date of filing: 07.10.2008
(51) Int. Cl.: A61K 39/09, A61P 31/04, C07K 16/12

(54) **New virulence factors of Streptococcus pneumoniae**

(71) Applicant: Stichting Katholieke Universiteit, more particularly the Radboud University Nijmegen Medical Centre, 6500 HB Nijmegen (NL)
(72) Inventor: The designation of the inventor has not yet been filed
(74) Representative: Hatzmann, Martin

(57) **Abstract**

The present invention provides proteins/genes, which are essential for survival, and consequently, for virulence of *Streptococcus pneumoniae in vivo*, and thus are ideal vaccine candidates for a vaccine preparation against pneumococcal infection. Further, also antibodies against said protein(s) are included in the invention.

## Description

### FIELD OF THE INVENTION

The invention relates to the field of medicine, more especially to the field of vaccines against bacterial infections, more particularly the genus *Streptococcus,* more particularly the species *Streptococcus pneumoniae.*

### BACKGROUND TO THE INVENTION

*Streptococcus pneumoniae* is the leading etiological agent of severe infections such as pneumonia, meningitis and sepsis. Young children, elderly and immunocompromised individuals are particularly vulnerable for pneumococcal diseases, which result in high morbidity and mortality (Hausdorff, W.P. et al., 2005, Lancet Infect. Dis. 5:83-93). The currently available vaccines against pneumococcal infections are based on serotype-specific capsular polysaccharides. These include a vaccine containing solely polysaccharides of 23 serotypes and a conjugate vaccine consisting of polysaccharides of the 7 most prevalent paediatric serotypes conjugated to an immunogenic carrier protein. The latter vaccine was introduced for the use in children under the age of 5, since their immune response to pure polysaccharides is inadequate. The introduction of this conjugate vaccine in the national vaccination program in the United States has had a major effect on invasive pneumococcal disease incidence (Whitney, C.G. et al., 2003, N. Eng. J. Med. 348:1737-1746).

Since at least 90 different polysaccharide structures are currently known within the species, polysaccharide-based vaccines only protect against a limited number of serotypes and hence, replacement by non-vaccine serotypes remains a threat for vaccine efficacy (Bogaert, D. et al., 2005, J. Clin. Microbiol. 43:74-83). Further, high production costs of the conjugate vaccines make their use in developing countries less feasible.

Treatment of *Streptococcus pneumoniae* infections is also impeded by the rise of strains resistant to the most commonly applied antibiotics (Levy, S.B. and Marshall, B., 2004, Nat. Med. 10:5122-5129). The development of an affordable effective vaccine against invasive pneumococcal disease in; especially, young children and elderly will have major benefits in terms of reducing disease burden and health care costs in both developed and developing countries. Immunogenic antigens of pneumococcal origin that are conserved amongst numerous serotypes would be desirable for conferring protection against infections caused by a broad range of serotypes. Much research effort is currently invested in search for pneumococcal proteins with protective potential to be included in future vaccines.

Methods searching for surface proteins of *Streptococcus pneumoniae* have been described (e.g.WO 98/18930), other methods have used immunological approaches to find possible antigenic determinants (WO 01/12219). On a genetic level, several methods have been used to determine which genes are needed by *Streptococcus pneumoniae* in the various niches it occupies in the host (conditionally essential genes) such as transcriptome analysis (Orihuela, C.J. et al., 2004, Infect. Immun. 72:4766-4777), differential fluorescence induction (Marra, A. et al., 2002, Infect. Immun. 70:1422-1433) and signature-tagged mutagenesis (Hava, D.L. and Camilli, A., 2002, Mol. Microbiol. 45:1389-1406; Lau, G.W. et al., 2001, Mol. Microbiol. 40:555-571; Polissi, A. et al., 1998, Infect. Immun. 66:5620-5629; Chen et al., 2008, PLoS ONE 3:e2950). Through these and other methods, several pneumococcal proteins have been identified and further investigated as potential vaccine candidates, such as the toxoid derivative of pneumolysin (PdB) (Briles, D.E. et al., 2003, J. Infect. Dis. 188:339-348; Ogunniyi, A.D. et al., 2000, Infect. Immun. 68:3028-3033; Ogunniyi, A.D. et al., 2001, Infect. Immun. 69:5997-6003), pneumococcal surface protein A (PspA) (Briles, D.E. et al., 2003, *supra*; Briles, D.E. et al., 2000, Infect. Immun. 68:796-800; Swiatlo, E. et al., 2003, Infect. Immun. 2003, 71:7149-7153; Wu, H.Y. et al., 1997, J. Infect. Dis. 175:839-846), pneumococcal surface adhesion A (PsaA) (Briles, D.E. et al., 2000, *supra*), choline binding protein A (CbpA) (Ogunniyi, A.D. et al., 2000, *supra*), BVH-3 (Hamel, J. et al., 2004, Infect. Immun. 72:2659-2670), PiuA and PiaA (Brown, J.S. et al., 2001, Infect. Immun. 69:6702-6706), pneumococcal protective protein A (PppA) (Green, B.A. et al., 2005, Infect. Immun. 73:981-989), putative proteinase maturation protein A (PpmA) (Adrian, P.V. et al., 2004, Vaccine 22:2737-2742; Overweg, K. et al., 2000, Infect. Immun. 68:4180-4188), IgA1 protease (IgA1) (Weiser, J.N. et al., 2003, Proc. Natl. Acad. Sci. USA 100:4215-4220) and the streptococcal lipoprotein rotamase A (SlrA) (Adrian, P.V. et al. *supra*).

Yet, there is still need for new vaccine candidates.

### SUMMARY OF THE INVENTION

The inventors now have found several proteins/genes of *Streptococcus pneumoniae* which are essential for the virulence of the pathogen, and which thus would be applicable in a vaccine for combating pneumococcal infections.

Accordingly, the invention comprises a vaccine formulation providing protection against pneumococcal infection in a subject, said formulation comprising an effective amount of a protein encoded by a gene listed in Table 1 and/or Table 2 or a functional homologue or an immunogenic part thereof together with at least one of a pharmaceutically acceptable diluent, carrier, excipient or adjuvant therefore. Preferably said immunogenic part is antigenic determinant of said pathogen. The protein of said formulation is preferably encoded by a gene listed in Table 1A, Table 1B, Table 2A, Table 2B, while most preferably said protein is encoded by a gene listed in two or more of Table 1A or Table 1B, Table 2A or Table 2B and the genes listed in International Patent Application PCT/NL2008/050191 (summarised in Table 3).

Further comprised in the invention is a formulation according to the above formulations, wherein said formulation provides protections against pneumonia, meningitis, otitis media and/or sepsis caused by *Streptococcus pneumoniae*.

In another embodiment, the invention comprises a protein encoded by a gene listed in Table 1 and/or 2 or an immunogenic part thereof, for use as a vaccine.

In another embodiment the invention comprises an antibody against a protein encoded by a gene listed in Table 1 and/or 2 or fragment thereof, preferably a humanized antibody or fragment thereof. Preferably said antibody or fragment thereof, preferably a humanized antibody or fragment thereof is for use as a medicament for the prophylactic or therapeutic treatment of a pneumococcal infection in a subject.

In yet another embodiment the invention comprises the use of said antibody or fragment thereof, preferably said humanized antibody or fragment thereof for the manufacture of a medicament for the prophylactic or therapeutic treatment of a pneumococcal infection in a subject.

Also comprised in the invention is a pharmaceutical composition comprising said antibody or fragment thereof, preferably said humanized antibody or fragment thereof, and a pharmaceutically acceptable carrier.

Further comprised in the invention is a method for prophylactic or therapeutic treatment of a pneumococcal infection in a subject comprising administering to a subject in need of such treatment an effective amount of a vaccine formulation as defined above and/or an effective amount of a pharmaceutical composition as defined above.

In another embodiment the invention comprises a method for preparing a pneumococcal vaccine formulation, the said method comprising bringing into association, an effective amount of a protein encoded by a gene listed in Table 1 and/or Table 2 or an immunogenic part thereof and at least one of a pharmaceutically acceptable diluent, carrier, excipient or adjuvant therefore. Preferably, said method comprises bringing into association, an effective amount of an antibody, preferably a humanized antibody, or fragment thereof, as described above and a pharmaceutically acceptable carrier.

### LEGENDS TO THE FIGURES

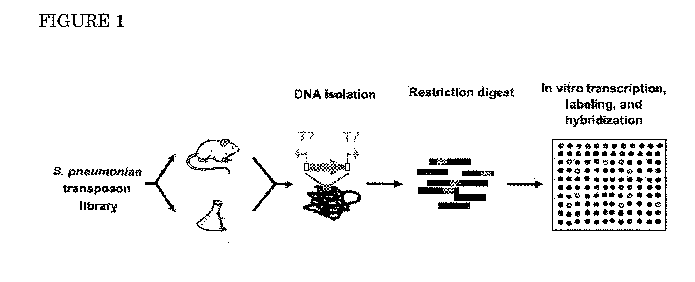

**Fig. 1** shows a schematic representation of the GAF procedure. A large *Streptococcus pneumoniae* transposon library is grown under nonselective and selective conditions. Subsequently, chromosomal DNA containing transposon (grey rectangle) with outward-facing T7 RNA polymerase promoters (arrow with T7) is isolated from each population. The DNA is digested, and the DNA adjacent to the transposon insertion site is amplified using *in vitro* transcription with T7 RNA polymerase. The RNA is used in standard procedures for microarray probe synthesis. Co-hybridization of probes derived from non-selective and selective conditions to a microarray will reveal which genes were disrupted in the mutants that disappeared during selection: only material derived from the nonselective condition will hybridise to those spots (grey spots).

### DETAILED DESCRIPTION

A "virulence factor" is referred to herein as a property of a pathogen that allows it to colonize and survive in the host, and consequently to cause disease. Virulence factors may distinguish a pathogenic micro-organism from otherwise identical non-pathogenic micro-organisms by allowing pathogens to invade, adhere to, and/or colonize a host, and then harm the host, as for an organism to be pathogenic it must be able to invade a host, multiply in the host, evade host defences, and harm the host in some way. As used herein the gene product of the genes of Table 1 and 2 are virulence factors.

The terms "invade" and "invasion" refer to the growing of infections into tissues, i.e., through and then beneath epithelial tissues, in particular it encompasses to the process of passage of mucosal tissue, either in the nasopharyngeal tissue or in the lungs, to the lymph fluid, the blood and/or the meningi. Thus, it encompasses both nasopharyngeal colonization and dissemination to the blood/meningi.

The term "functional fragment" refers to a shortened version of the protein, which is a functional variant or functional derivative. A "functional variant" or a "functional derivative" of a protein is a protein the amino acid sequence of which can be derived from the amino acid sequence of the original protein by the substitution, deletion and/or addition of one or more amino acid residues in a way that, in spite of the change in the amino acid sequence, the functional variant retains at least a part of at least one of the biological activities of the original protein that is detectable for a person skilled in the art. A functional variant is generally at least 60% homologous (preferably the amino acid sequence is at least 60% identical), advantageously at least 70% homologous and even more advantageously at least 80 or 90% homologous to the protein from which it can be derived. A functional variant may also be any functional part of a protein; the function in the present case being particularly but not exclusively essential activity for blood or cerebrospinal fluid colonization. "Functional" as used herein means functional in *Streptococcus pneumoniae* bacteria and capable of eliciting antibodies which give protection against disease caused by said bacteria.

The expression "conservative substitutions" as used with respect to amino acids relates to the substitution of a given amino acid by an amino acid having physicochemical characteristics in the same class. Thus where an amino acid of the protein encoded by the genes listed in Tables 1 and 2 has a hydrophobic characterising group, a conservative substitution replaces it by another amino acid also having a hydrophobic characterising group; other such classes are those where the characterising group is hydrophilic, cationic, anionic or contains a thiol or thioether. Such substitutions are well known to those of ordinary skill in the art, i.e. see US 5,380,712. Conservative amino acid substitutions may be made, for example within the group of aliphatic nonpolar amino acids (Gly, Ala, Pro, Ile, Leu, Val), the group of polar uncharged amino acids (Cys, Ser, Thr, Met, Asn, Gln), the group of polar charged amino acids (Asp, Glu, Lys, Arg) or the group of aromatic amino acids (His, Phe, Tyr, Trp).

The term " immunogenic part" includes reference to any part of a protein encoded by the genes listed in Tables 1 and 2, or a functional homologue or functional fragment thereof, which is capable of eliciting an immune response in a mammal. Said immunogenic part preferably corresponds to an antigenic determinant of said pathogen.

As used herein, the term "antigen" refers to a molecule capable of being bound by an antibody or a T cell receptor (TCR) if presented by molecules of the major histocompatibility complex (MHC). The term "antigen", as used herein, also encompasses T-cell epitopes. A T-cell epitope is recognized by a T-cell receptor in the context of a MHC class I, present on all cells of the body except erythrocytes, or class II, present on immune cells and in particular antigen presenting cells. This recognition event leads to activation of T-cells and subsequent effector mechanisms such as proliferation of the T-cells, cytokine secretion, perforin secretion etc. An antigen is additionally capable of being recognized by the immune system and/or being capable of inducing a humoral immune response and/or cellular immune response leading to the activation of B- and/or T-lymphocytes. This may, however, require that, at least in certain cases, the antigen contains or is linked to a T-Helper cell epitope and is given in adjuvant. An antigen can have one or more epitopes (Band T-epitopes). The specific reaction referred to above is meant to indicate that the antigen will preferably react, typically in a highly selective manner, with its corresponding antibody or TCR and not with the multitude of other antibodies or TCRs which may be evoked by other antigens. Antigens as used herein may also be mixtures of several individual antigens. Antigens, as used herein, include infectious disease antigens, more especially antigens of *Streptococcus pneumoniae,* more preferable antigens derived from the proteins encoded by the genes listed in Tables 1 and 2 and fragments and derivatives thereof. Furthermore, antigens used for the present invention can be peptides, proteins, domains, or lipids, especially those lipids that are associated to the proteins encoded by the genes listed in Tables 1 and 2 as lipoproteins.

As used herein, the term "antigenic determinant" is meant to refer to that portion of an antigen that is specifically recognized by either B- or T-lymphocytes. B-lymphocytes respond to foreign antigenic determinants via antibody production, whereas T-lymphocytes are the mediator of cellular immunity. Thus, antigenic determinants or epitopes are those parts of an antigen that are recognized by antibodies, or in the context of an MHC, by T-cell receptors. An antigenic determinant may contain one or more epitopes. Epitopes may be present on the intracellular (inside), transmembrane spanning (transmembrane), as well as extracellular (outside) regions of a protein molecule. It is expected that antigenic determinants are associated with in particular those regions of the surface proteins encoded by the genes listed in Tables 1A, 1B, 2A, and 2B which are on the outside of the cytoplasmic membrane of the bacteria. These regions can be predicted from the sequences as provided, by using for instance one of the software programs SignalP3.0, PSORTb or TMHMM, e.g. version 2.0c, which provides a method for prediction transmembrane helices based on a hidden Markov model.

The term "prophylactic or therapeutic treatment of an infection by *Streptococcus pneumoniae*" or "prophylactic or therapeutic treatment of a pneumococcal infection" refers to both prophylactic or therapeutic treatments wherein virulence of the pathogen is blocked or diminished, but also to treatments wherein antibodies against any of the proteins encoded by the genes listed in Table 1 or 2 recognize the bacteria and will protect the host against infection, either directly through immune clearance, or indirectly by blocking the activity of the protein, thereby inhibiting the growth of the bacteria. Also, the term refers to blocking the function of any of the proteins encoded by the genes listed in Tables 1 and 2 *in vivo* thereby reducing the adhesion abilities of the pathogen with a concomitant reduction in colonization and invasion capabilities. The term thus includes inducing immune responses in subjects using vaccine formulations of the invention, as well as inhibiting growth of the pathogen *in vivo* by using antibodies of the present invention as an active compound in a pharmaceutical composition administered to the subject. Also included are the inhibition of the virulence and/or growth of the bacteria by treatment with antibiotics.

The term "antibody" refers to molecules which are capable of binding an epitope or antigenic determinant and includes reference to antigen binding forms of antibodies (e. g., Fab, F(ab)2). The term "antibody" frequently refers to a polypeptide substantially encoded by an immunoglobulin gene or immunoglobulin genes, or fragments thereof which specifically bind and recognize an analyte (antigen). However, while various antibody fragments can be defined in terms of the digestion of an intact antibody, one of skill will appreciate that such fragments may be synthesized de novo either chemically or by utilizing recombinant DNA methodology. Thus, the term antibody, as used herein, also includes antibody fragments such as single chain Fv, chimeric antibodies (i. e., comprising constant and variable regions from different species), humanized antibodies (i. e., comprising a complementarity determining region (CDR) from a non-human source) and heteroconjugate antibodies (e. g., bispecific antibodies). The antibody can be monoclonal or polyclonal and can be prepared by techniques that are well known in the art such as immunization of a host and collection of sera (polyclonal) (see, e.g., Parker, Radioimmunoassay of Biologically Active Compounds, Prentice-Hall (Englewood Cliffs, N.J., U.S., 1976), Butler, J. Immunol. Meth. 7, 1-24 (1975); Broughton and Strong, Clin. Chem. 22, 726-732 (1976); and Playfair, et al., Br. Med. Bull. 30, 24-31 (1974)) or by preparing continuous hybrid cell lines and collecting the secreted protein (monoclonal) (see, *e*.*g*., Kohler et al in Nature 256, 495-497 (1975) and Eur. J. Immunol. 6, 511-519 (1976); by Milstein et al. Nature 266, 550-552 (1977); and by Walsh Nature 266, 495 (1977)) or by cloning and expressing nucleotide sequences or mutagenized versions thereof coding at least for the amino acid sequences required for specific binding of natural antibodies. Antibodies may include a complete immunoglobulin or fragment thereof, which immunoglobulins include the various classes and isotypes, such as IgA, IgD, IgE, IgG1, IgG2a, IgG2b and lgG3, IgM, etc. Fragments thereof may include Fab, Fv and F(ab')2, Fab', and the like. In addition, aggregates, polymers, and conjugates of immunoglobulins or their fragments can be used where appropriate so long as binding affinity for a particular molecule is maintained.

As used herein, the term "monoclonal antibody" refers to an antibody composition having a homogeneous antibody population. The term is not limited regarding the species or source of the antibody, nor is it intended to be limited by the manner in which it is made. The term encompasses whole immunoglobulins as well as fragments such as Fab, F(ab')2, Fv, and others, such as CDR fragments, which retain the antigen binding function of the antibody. Monoclonal antibodies of any mammalian species can be used in this invention. In practice, however, the antibodies will typically be of rat or murine origin because of the availability of rat or murine cell lines for use in making the required hybrid cell lines or hybridomas to produce monoclonal antibodies.

As used herein, the term "humanized monoclonal antibodies" means that at least a portion of the exposed amino acids in the framework regions of the antibody (or fragment), which do not match with the corresponding amino acids in the most homologous human counterparts, are changed, such as by site directed mutagenesis of the DNA encoding the antibody. Because these exposed amino acids are on the surface of the molecule, this technique is called "resurfacing." Moreover, because the amino acids on the surface of the molecule are the ones most likely to give rise to an immune response, this resurfacing decreases the immunogenicity of the monoclonal antibody when administered to a species whose cell line was not used to generate the antibody, such as a human. The term "humanized monoclonal antibody" also includes chimeric antibody wherein the light and heavy variable regions of a monoclonal antibody generated by a hybridoma from a non-human cell line are each attached, via recombinant technology, to one human light chain constant region and at least one heavy chain constant region, respectively. The preparation of such chimeric (i.e. humanized) antibodies is well known in the art.

The term "specifically recognizing", includes reference to a binding reaction between an antibody and a protein having an epitope recognized by the antigen binding site of the antibody. This binding reaction is determinative of the presence of a protein having the recognized epitope amongst the presence of a heterogeneous population of proteins and other biologics. Specific binding to an antibody under such conditions may require an antibody that is selected for its specificity for a particular protein. For example, antibodies raised to the proteins encoded by the genes listed in Tables 1 and 2 of the present invention can be selected to obtain antibodies specifically recognizing said proteins. The proteins used as immunogens can be in native conformation or denatured so as to provide a linear epitope. A variety of immunoassay formats may be used to select antibodies specifically recognizing a particular protein (or other analyte). For example, solid-phase ELISA immunoassays are routinely used to select monoclonal antibodies specifically immunoreactive with a protein. See Harlow and Lane, Antibodies, A Laboratory Manual, Cold Spring Harbor Publications, New York (1988), for a description of immunoassay formats and conditions that can be used to determine selective reactivity.

A "subject" as referred to herein is meant to include mammals and other animals, wherein mammals include for example, humans, apes, monkeys, horses, cattle, pigs, goats, dogs, cats, rats, mice, and sheep. The term "non-human animal" is meant to disclaim humans. Preferably in the present invention, the subject is a human, more preferably a child or an elderly person.

The virulence proteins/genes of the present invention have been identified by genomic array footprinting (GAF), which is a high-throughput method to identify conditionally essential gene in *Streptococcus pneumoniae* by using a combination of random transposon mutagenesis and microarray technology (see Bijlsma, J.J.E. et al., 2007, Appl. Environm. Microbiol. 73(5):1514-1524). GAF detects the transposon insertion sites in a mutant library by amplifying and labelling the chromosomal DNA adjacent to the transposon and subsequent hybridisation of these probes to a microarray. Identification of transposon insertion sites in mutants that have disappeared from the library due to selection, which represent conditionally essential genes, is achieved by differential hybridisation of the probes generated from the library grown under two conditions to an array. For specific detection of essential genes for survival in the blood, mutant libraries of *Streptococcus pneumoniae* (prepared as described in the experimental part) were used to infect mice in a murine bacteraemia model of infection. After challenge mutants were identified that had disappeared from the blood samples taken from the mice, and the disrupted genes of these mutants were identified. For specific detection of genes essential for survival in cerebrospinal fluid (CSF), mutant libraries of *Streptococcus pneumoniae* (prepared as described in the experimental part) were used to infect rabbits in a rabbit meningitis model of infection. After challenge mutants were identified that had disappeared from the CSF samples taken from the rabbits, and the disrupted genes of these mutants were identified.

The genes found to be essential for survival in the blood in the bacteraemia model are provided in Table 1. Table 1A lists the genes which are predicted to be located at the surface based on their sequence (using various prediction servers, such as SignalP3.0 (http://www.cbs.dtu.dk/services/SignalP), and PSORTb (http://www.psort.org). Furthermore, Table 1A lists the genes, which are predicted to be surface localised, based on the following criteria:
- one to three predicted transmembrane helices (determined using TMHMM (http://www.ebs.dtu.dk/services/TMHMM)); or
- components IIC and IID of PTS systems. Table 1B lists the genes, which are predicted to be localised in the cytoplasm.

The genes found to be essential for survival in the CSF in the rabbit meningitis model are listed in Tables 2A-2B on the same criteria as for Table 1.

The surface-localised proteins of the genes of Tables 1 and 2 are especially preferred as a vaccine component according to the present invention.

Next to the genes listed in Tables 1 and 2, more genes (listed in Tables 3 and 4) have been identified in the current experimental set-up. The genes/proteins of Table 3 have been identified earlier as genes/proteins which would be suitable as vaccine candidates for *Streptococcus pneumoniae* using the GAF technology, as listed in International Patent Application PCT/NL2008/050191. The genes/proteins of Table 4 have been identified earlier as genes/proteins, which would be suitable as vaccine candidates for *Streptococcus pneumoniae,* evident from existing literature. The fact that these genes were found in our experiments emphasizes the usefulness of the methodology for finding potential vaccine candidates.

**Table 1A. Conditionally essential Streptococcus pneumoniae genes identified in blood in the bacteraemia model, which encode a predicted surface-localised protein. Locus indicates the gene number assigned by TIGR-CMG annotation (Tettelin H. et al., 2001, Science. 293:498-506; TIGR Comprehensive Microbial Resource database http://cmr.tigr.org/tigr-scripts/CMR/CmrHomePage.cgi).**

| **SP nr.** | **Annotation** | **Gene** | **Mainrole** | **# Time-points identified** |
|---|---|---|---|---|
| SP0249 | PTS system, IIB component | | Transport and binding proteins | 3 |
| SP0305 | PTS system, IIB component | | Transport and binding proteins | 2 |
| SP0389 | hypothetical protein | | Hypothetical proteins | 1 |
| SP0604 | sensor histidine kinase VncS | vncS | Signal transduction | 2 |
| SP0747 | hypothetical protein | | Hypothetical proteins | 2 |
| SP0904 | conserved hypothetical protein | | Hypothetical proteins-Conserved | 2 |
| SP0998 | hypothetical protein | | Hypothetical proteins | 2 |
| SP1108 | hypothetical protein | | Hypothetical proteins | 3 |
| SP1330 | N-acetylmannosamine-6-P epimerase, putative | nanE | Energy metabolism | 3 |
| SP1945 | hypothetical protein | | Hypothetical proteins | 2 |
| SP1967 | conserved hypothetical protein | | Hypothetical proteins-Conserved | 3 |
| SP2129 | PTS system, IIC component, putative | | Transport and binding proteins | 2 |

**Table 1B. Conditionally essential Streptococcus pneumoniae genes identified in blood in the bacteraemia model, which encode a predicted cytoplasm-localised protein.**

| **SP nr.** | **Annotation** | **Gene** | **Mainrole** | **# Time-points identified** |
|---|---|---|---|---|
| SP0059 | hypothetical protein | | Hypothetical proteins | 1 |
| SP0094 | hypothetical protein | | Hypothetical proteins. | 2 |
| SP0134 | hypothetical protein | | Hypothetical proteins | 2 |
| SP0147 | hypothetical protein | | Hypothetical proteins | 1 |
| SP0184 | hypothetical protein | | Hypothetical proteins | 1 |
| SP0192 | conserved hypothetical protein | | Hypothetical proteins-Conserved | 2 |
| SP0288 | conserved hypothetical protein | | Hypothetical proteins-Conserved | 3 |
| SP0313 | glutathione peroxidase | | Cellular processes | 2 |
| SP0388 | hypothetical protein, authentic frameshift | | Disrupted reading frame | 1 |
| SP0465 | hypothetical protein | | Hypothetical proteins | 2 |
| SP0476 | PTS system, lactose-specific IIA component | IacF | Transport and binding proteins | 2 |
| SP0504 | hypothetical protein | | Hypothetical proteins | 2 |
| SP0511 | hypothetical protein | | Hypothetical proteins | 2 |
| SP0513 | hypothetical protein | | Hypothetical proteins | 2 |
| SP0541 | bacteriocin BIpO | blpO | Cellular processes | 2 |
| SP0544 | immunity protein BlpX | blpX | Cellular processes | 2 |
| SP0573 | hypothetical protein | | Hypothetical proteins | 1 |
| SP0598 | hypothetical protein | | Hypothetical proteins | 3 |
| SP0639 | hypothetical protein | | Hypothetical proteins | 2 |
| SP0649 | conserved hypothetical protein, degenerate | | Disrupted reading frame | 1 |
| SP0650 | hypothetical protein | | Hypothetical proteins | 2 |
| SP0654 | hypothetical protein | | Hypothetical proteins | 1 |
| SP0666 | conserved hypothetical protein | | Hypothetical proteins-Conserved | 2 |
| SP0773 | hypothetical protein | | Hypothetical proteins | 1 |
| SP0776 | KH domain protein | | Unknown function | 2 |
| SP0833 | hypothetical protein | | Hypothetical proteins | 2 |
| SP0834 | hemolysin-related protein | | Unknown function | 2 |
| SP0906 | hypothetical protein | | Hypothetical proteins | 2 |
| | capsular polysaccharide biosynthesis protein, | | | |
| SP0907 | putative | | Cell envelope | 2 |
| SP0908 | transcriptional regulator, putative | | Regulatory functions | 2 |
| SP0926 | hypothetical protein | | Hypothetical proteins | 2 |
| | replication initiator protein, truncation, authentic | | | |
| SP0940 | frameshift | | DNA metabolism | 2 |
| SP1070 | conserved hypothetical protein | | Hypothetical proteins-Conserved | 2 |
| SP1099 | ribosomal large subunit pseudouridine synthase | | Protein synthesis | 3 |
| SP1114 | ABC transporter, ATP-binding protein | | Transport and binding proteins | 2 |
| SP1140 | hypothetical protein | | Hypothetical proteins | 2 |
| SP1141 | hypothetical protein | | Hypothetical proteins | 2 |
| SP1142 | hypothetical protein | | Hypothetical proteins | 2 |
| SP1146 | hypothetical protein | | Hypothetical proteins | 1 |
| SP1197 | conserved hypothetical protein | | Hypothetical proteins-Conserved | 2 |
| SP1211 | hypothetical protein | | Hypothetical proteins | 2 |
| SP1221 | type II restriction endonuclease, putative | | DNA metabolism | 2 |
| | 3-isopropylmalate dehydratase, small subunit, | | | |
| SP1255 | putative | | Amino acid biosynthesis | 2 |
| SP1261 | conserved hypothetical protein | | Hypothetical proteins-Conserved | 2 |
| SP1294 | crcB protein | crcB | Unknown function | 2 |
| SP1369 | prephenate dehydratase | pheA | Amino acid biosynthesis | 3 |
| SP1411 | conserved hypothetical protein | | Hypothetical proteins-Conserved | 2 |
| SP1415 | glucosamine-6-phosphate isomerase | nagB | Central intermediary metabolism | 2 |
| SP1436 | hypothetical protein | | Hypothetical proteins | 2 |
| SP1452 | hypothetical protein | | Hypothetical proteins | 2 |
| SP1473 | conserved hypothetical protein | | Hypothetical proteins-Conserved | 1 |
| SP1476 | hypothetical protein | | Hypothetical proteins | 2 |
| SP1548 | hypothetical protein | | Hypothetical proteins | 1 |
| SP1597 | conserved hypothetical protein | | Hypothetical proteins-Conserved | 2 |
| SP1611 | hypothetical protein | | Hypothetical proteins | 2 |
| SP1627 | conserved hypothetical protein | | Hypothetical proteins-Conserved | 1 |
| SP1669 | MutT/nudix family protein | | DNA metabolism | 2 |
| SP1756 | conserved domain protein | | Hypothetical proteins-Domain | 2 |
| SP1829 | galactose-1-phosphate uridylyltransferase | galT | Energy metabolism | 2 |
| SP1836 | hypothetical protein | | Hypothetical proteins | 2 |
| SP1865 | glutamyl-aminopeptidase | pepA | Protein fate | 2 |
| SP1868 | conserved domain protein | | Hypothetical proteins-Domain | 1 |
| | oligopeptide ABC transporter, ATP-binding | | | |
| SP1887 | protein | amiF | Transport and binding proteins | 2 |
| SP1907 | chaperonin, 10 kDa | groES | Protein fate | 2 |
| SP1911 | thioredoxin, putative | | Energy metabolism | 2 |
| SP1934 | hypothetical protein | | Hypothetical proteins | 3 |
| | type II restriction-modification system regulatory | | | |
| SP1936 | protein | | Regulatory functions | 1 |
| SP1982 | conserved hypothetical protein | | Hypothetical proteins-Conserved | 2 |
| SP1983 | ribulose-phosphate 3-epimerase | rpe | Energy metabolism | 2 |
| SP2004 | hypothetical protein | | Hypothetical proteins | 1 |
| SP2045 | conserved hypothetical protein | | Hypothetical proteins-Conserved | 2 |
| SP2055 | alcohol dehydrogenase, zinc-containing | | Energy metabolism | 3 |
| SP2061 | conserved hypothetical protein | | Hypothetical proteins-Conserved | 1 |
| SP2071 | conserved domain protein | | Hypothetical proteins-Domain | 1 |
| SP2104 | hypothetical protein | | Hypothetical proteins | 2 |
| SP2109 | maltodextrin ABC transporter, permease protein | malC | Transport and binding proteins | 2 |
| SP2141 | glycosyl hydrolase-related protein | | Unknown function | 2 |
| SP2166 | L-fuculose phosphate aldolase | fucA | Energy metabolism | 2 |
| SP2174 | D-alanyl carrier protein | dltC | Cell envelope | 2 |
| SP2195 | transcriptional regulator CtsR | ctsR | Regulatory functions | 2 |
| SP2196 | ABC transporter, ATP-binding protein | | Transport and binding proteins | 2 |
| SP2198 | ABC transporter, permease protein | | Transport and binding proteins | 3 |
| SP2200 | hypothetical protein | | Hypothetical proteins | 1 |
| SP2237 | competence stimulating peptide 2 | comC2 | Cellular processes | 1 |

**Table 2A. Conditionally essential Streptococcus pneumoniae genes identified in CSF in the meningitis model, which encode a predicted surface-localised protein.**

| **SP nr.** | **Annotation** | **Gene** | **Mainrole** | **# Time-points identified** |
|---|---|---|---|---|
| SP1330 | N-acetylmannosamine-6-P epimerase, putative | nanE | Energy metabolism | 2 |

**Table 2B. Conditionally essential Streptococcus pneumoniae genes identified in CSF in the meningitis model, which encode a predicted cytoplasm-localised protein.**

| **SP nr.** | **Annotation** | **Gene** | **Mainrole** | **# Time-points identified** |
|---|---|---|---|---|
| SP0019 | adenylosuccinate synthetase | purA | Purines, pyrimidines, nucleosides, nucleotides | 2 |
| SP0031 | hypothetical protein | | Hypothetical proteins | 1 |
| SP0649 | conserved hypothetical protein, degenerate | | Disrupted reading frame | 1 |
| SP1005 | conserved domain protein, degenerate | | Disrupted reading frame | 1 |
| | type II DNA modification methyltransferase | | | |
| SP1336 | Spn5252IP | | DNA metabolism | 2 |
| SP1356 | Atz/Trz family protein | | Unknown function | 2 |
| SP1635 | hypothetical protein | | Hypothetical proteins | 2 |
| SP2198 | ABC transporter, permease protein | | Transport and binding proteins | 2 |

**Table 3. Genes found in the bactaeremia and meningitis GAF screens that have been identified in previous in vivo GAF screens, listed in International Patent Application PCT/NL2008/050191.**

| | **Genome-wide screen in which gene was identified:** | | **International Patent Application** PCT/NL2008/050191 **infection model in which gene was identified:** | | | |
|---|---|---|---|---|---|---|
| **SP nr.** | **Bacteraemia** | **Meningitis** | **Pneumonia-NPL** | **Pneumonia-blood** | **Colonization** | **Bacteraemia** |
| SP0018 | X | | | | X | |
| SP0029 | X | X | | X | X | X |
| SP0058 | X | X | X | X | X | X |
| SP0062 | X | | | X | | X |
| SP0064 | X | | | | X | |
| SP0067 | X | X | | X | X | X |
| SP0072 | X | | | | X | |
| SP0079 | X | X | | | X | |
| SP0098 | | X | | | | X |
| SP0099 | | X | | X | | X |
| SP0101 | X | X | | | X | |
| SP0116 | X | | X | X | X | |
| SP0138 | X | | | X | | |
| SP0139 | X | | | X | | |
| SP0152 | | X | | | X | |
| SP0158 | X | | | | X | |
| SP0197 | X | X | | | X | |
| SP0206 | X | | X | X | X | |
| SP0207 | X | | | | X | |
| SP0245 | X | | X | X | X | |
| SP0276 | X | X | | | X | |
| SP0279 | X | X | | | X | |
| SP0282 | | X | X | X | | X |
| SP0287 | X | | | X | | |
| SP0302 | X | | X | X | X | |
| SP0309 | X | | | | X | |
| SP0340 | X | | X | X | | X |
| SP0341 | X | | | | | X |
| SP0342 | X | X | | | X | X |
| SP0416 | | X | | | X | |
| SP0446 | X | | X | | | X |
| SP0470 | X | | | X | X | |
| SP0507 | X | | | X | X | |
| SP0514 | X | | X | X | X | |
| SP0521 | X | | X | X | X | X |
| SP0534 | X | | | | X | |
| SP0540 | X | | | X | | |
| SP0546 | X | | | | X | |
| SP0552 | | X | | | X | |
| SP0585 | X | X | X | | X | X |
| SP0597 | X | | | | X | |
| SP0621 | X | | | | X | |
| SP0634 | X | | X | | | |
| SP0635 | X | | | X | X | X |
| SP0646 | X | | | | X | |
| SP0651 | X | | X | | X | |
| SP0668 | X | | | | | X |
| SP0679 | X | | | X | X | X |
| SP0695 | | X | X | | X | |
| SP0696 | X | | X | X | X | |
| SP0698 | X | | | X | X | |
| SP0705 | X | | X | | | |
| SP0718 | X | | | | X | |
| SP0722 | X | | | X | | |
| SP0731 | X | X | | | X | |
| SP0748 | X | X | X | | X | |
| SP0749 | X | X | X | X | | |
| SP0751 | | X | X | | X | |
| SP0752 | X | X | X | | X | |
| SP0753 | X | X | X | | | |
| SP0754 | X | | X | | X | |
| SP0768 | X | | X | | | X |
| SP0792 | X | | | X | X | X |
| SP0810 | X | | | | X | X |
| SP0822 | X | X | | | X | |
| SP0826 | X | X | | | X | |
| SP0843 | X | | | X | X | |
| SP0861 | X | | | | X | |
| SP0881 | X | X | X | X | X | X |
| SP0888 | X | | | | X | |
| SP0893 | X | | X | X | | X |
| SP0901 | X | | | X | X | |
| SP0925 | X | X | | X | | X |
| SP0949 | X | | X | X | X | X |
| SP0962 | X | | X | | X | |
| SP1011 | X | | X | X | X | X |
| SP1025 | | X | X | X | X | X |
| SP1050 | X | | | X | X | X |
| SP1051 | X | | | | X | X |
| SP1052 | X | | X | X | X | |
| SP1053 | X | | X | X | X | X |
| SP1059 | X | X | | | X | |
| SP1060 | X | | | | X | |
| SP1062 | | X | | | X | |
| SP1063 | | X | | | X | |
| SP1069 | X | X | | | X | |
| SP1096 | X | | X | | X | |
| SP1097 | X | | | | X | |
| SP1105 | X | | | | X | |
| SP1138 | X | | | X | X | |
| SP1139 | X | | | X | | |
| SP1177 | X | | X | X | | |
| SP1178 | X | | | X | X | |
| SP1186 | X | | | X | X | |
| SP1189 | X | | | | X | |
| SP1192 | X | | | X | | X |
| SP1209 | X | | | X | X | |
| SP1210 | X | | | X | X | X |
| SP1215 | X | | | X | | X |
| SP1218 | X | | | | X | |
| SP1224 | X | | | | X | |
| SP1235 | X | | X | | | |
| SP1245 | X | | X | X | X | X |
| SP1259 | X | | | | X | X |
| SP1284 | X | | | | X | |
| SP1296 | X | X | | | X | |
| SP1297 | X | X | | | X | |
| SP1298 | X | X | X | | X | |
| SP1299 | X | X | X | X | X | X |
| SP1302 | X | | | | X | |
| SP1311 | X | | X | | | |
| SP1322 | X | | | X | X | X |
| SP1323 | X | | | X | X | X |
| SP1327 | X | | | | X | |
| SP1331 | X | X | | X | | X |
| SP1332 | X | | | X | | |
| SP1333 | X | | X | X | | X |
| SP1340 | X | | | | X | |
| SP1349 | X | | | X | X | X |
| SP1353 | X | | | | X | |
| SP1368 | X | | X | X | | X |
| SP1376 | | X | X | | X | |
| SP1379 | X | | | | X | |
| SP1392 | X | | | | X | |
| SP1393 | X | X | X | X | X | X |
| SP1394 | X | | | | X | |
| SP1397 | X | | X | X | X | X |
| SP1430 | X | | X | | X | |
| SP1462 | | X | | | X | |
| SP1465 | X | X | X | X | X | |
| SP1466 | X | X | | | X | |
| SP1494 | X | | | | X | |
| SP1495 | X | | | X | X | |
| SP1502 | X | X | | X | X | |
| SP1537 | X | | X | X | X | X |
| SP1563 | | X | X | X | X | X |
| SP1567 | X | | X | | X | |
| SP1609 | X | | X | | X | |
| SP1618 | X | | | | X | |
| SP1620 | X | | | X | X | X |
| SP1626 | X | | X | | X | |
| SP1628 | X | | | | X | |
| SP1630 | X | | | X | | X |
| SP1639 | X | | | X | X | |
| SP1643 | X | | X | X | | |
| SP1680 | X | | | | X | |
| SP1681 | X | | | | X | |
| SP1690 | X | | X | X | X | X |
| SP1691 | X | | X | | X | |
| SP1704 | X | | X | | X | |
| SP1718 | X | | | X | X | |
| SP1728 | X | | | X | X | X |
| SP1730 | X | | | | X | |
| SP1740 | X | | X | | | |
| SP1741 | X | | X | | | |
| SP1743 | X | | X | | | |
| SP1751 | X | | | | X | |
| SP1759 | X | | | X | X | |
| SP1762 | X | | X | | . | |
| SP1765 | X | | | | X | |
| SP1801 | X | | X | X | X | X |
| SP1806 | X | | | | X | |
| SP1810 | X | | | X | X | |
| SP1822 | X | | | X | X | |
| SP1831 | X | | X | | X | |
| SP1851 | X | | | X | X | X |
| SP1863 | X | | | X | X | X |
| SP1864 | X | | | X | X | X |
| SP1908 | X | | | | X | |
| SP1917 | X | | | | X | |
| SP1931 | X | X | X | X | X | |
| SP1944 | X | | | | X | |
| SP1946 | X | | | | X | |
| SP1947 | X | | | | X | |
| SP1955 | X | | X | X | X | |
| SP1963 | X | | | X | X | X |
| SP1966 | X | X | | | | X |
| SP1974 | X | | | | X | |
| SP1979 | X | | | X | X | |
| SP1995 | X | X | | | X | |
| SP1996 | X | | | | X | |
| SP2021 | X | X | | X | X | X |
| SP2035 | X | | | | X | |
| SP2044 | X | | | | X | |
| SP2062 | X | | | | X | |
| SP2077 | X | | | | X | |
| SP2084 | X | | X | X | X | X |
| SP2088 | X | | | X | X | |
| SP2090 | X | | | X | X | X |
| SP2094 | X | | | | X | |
| SP2096 | X | | X | X | X | X |
| SP2102 | X | | | | X | |
| SP2115 | X | | | X | X | |
| SP2117 | X | | | | X | |
| SP2120 | X | | | | X | |
| SP2123 | X | | | X | | X |
| SP2135 | X | | X | X | | X |
| SP2147 | X | | X | X | | |
| SP2151 | X | | | X | X | X |
| SP2183 | X | | | | X | |
| SP2186 | X | | X | | | |
| SP2197 | X | | X | X | | X |
| SP2205 | X | X | X | X | X | X |
| SP2206 | X | X | X | X | X | X |
| SP2208 | X | | | | X | |
| SP2209 | X | | X | | X | |
| SP2229 | X | | | X | X | |
| SP2233 | X | | X | X | | X |

**Table 4. Genes found in the bactaeremia and meningitis GAF screens that have been identified in literature as potential vaccine candidates. Indicated literature references are: H: Hava et al., 2002, Mol. Microbiol. 45:1389-1406; L: Lau et al., 2001, Mol. Microbiol. 40:555-571; P: Polissi et al., 1998, Infect. Immun. 66:5620-5629; C: Chen et al., 2008, PLoS ONE 3:e2950; 1: Throup et al., 2000, Mol. Microbiol. 35:566-576; 2: Zysk G. et al., 2000, Infect Immun. 68:3740-3743; 3: Caimano, M.J. et al., in: Streptococcus pneumoniae - Molecular biology and mechanisms of disease, A. Tomasz (Ed.), Mary Ann Liebert, Larchmont, NY, 2000, p.115.; 4: Tong et al., 2000, Infect. Immun. 68:921-924; 5: Iyer R. and Camilli A., 2007, Mol. Microbiol. 66:1-13; 6: Orihuela et al., 2004, Infect. Immun. 72:5582-5596; 7: Brown, J.S. et al., 2001, Infect. Immun. 69:6702-6706; 8: Marra, A. et al., 2002, Infect. Immun. 70:1422-1433; 9: Dintilhac, A. et al., 1997, Res. Microbiol. 148:119-131; 10: Kwon et al., 2003, Infect. Immun. 71:3757-3765; 11: Bartilson, M. et al., 2001, Mol. Microbiol. 39:126-135**

| | | Infection model in which gene was identified: | | |
|---|---|---|---|---|
| SP nr. | Annotation | Bactaeremia | Meningitis | Ref |
| SP0042 | competence factor transporting ATP-binding/permease protein | X | X | C |
| SP0044 | phosphoribosylaminoimidazole-succinocarboxamide synthase | X | | P |
| SP0046 | amidophosphoribosyltransferase | X | | C |
| SP0049 | vanZ protein, putative | X | | H |
| SP0063 | PTS system, IID component | X | | H |
| SP0081 | glycosyl transferase, family 2, authentic point mutation | X | | C |
| SP0084 | sensor histidine kinase | X | | 1 |
| SP0095 | conserved hypothetical protein | X | | H |
| SP0100 | conserved hypothetical protein | X | | H,C |
| SP0149 | lipoprotein | | X | C |
| SP0151 | ABC transporter, ATP-binding protein | | X | C |
| SP0157 | hypothetical protein | X | | H |
| SP0160 | conserved domain protein | X | | H |
| SP0175 | 6,7-dimethyl-8-ribityllumazine synthase | X | | 2 |
| SP0198 | hypothetical protein | X | X | H |
| SP0199 | cardiolipin synthetase | | X | H |
| SP0246 | transcriptional regulator, DeoR family | X | | H |
| SP0267 | oxidoreductase, putative | X | | P,H |
| SP0308 | PTS system, IIA component | X | X | C |
| SP0314 | hyaluronidase | X | | H |
| SP0323 | PTS system, IIB component | X | | C |
| SP0348 | capsular polysaccharide biosynthesis protein Cps4C | | X | 3 |
| SP0349 | capsular polysaccharide biosynthesis protein Cps4D | | X | 3 |
| SP0350 | capsular polysaccharide biosynthesis protein Cps4E | X | X | 3 |
| SP0351 | capsular polysaccharide biosynthesis protein Cps4F | X | X | 3 |
| SP0358 | capsular polysaccharide biosynthesis protein cps4J | X | X | 3 |
| SP0396 | PTS system, mannitol-specific IIA component | X | | H |
| SP0490 | hypothetical protein | X | | C |
| SP0494 | GTP synthase | X | X | H |
| SP0603 | DNA-binding response regulator VncR | X | | C |
| SP0633 | hypothetical protein | X | | H |
| SP0645 | PTS system IIA component, putative | X | | H |
| SP0648 | beta-galactosidase | X | | H,C |
| SP0665 | chorismate binding enzyme | X | X | H |
| SP0723 | conserved domain protein | X | | C |
| SP0728 | hypothetical protein | X | | H |
| SP0746 | ATP-dependent Clp protease, proteolytic subunit | | X | 10 |
| SP0823 | amino acid ABC transporter, permease protein | | X | L |
| SP0829 | phosphopentomutase | X | | H,C |
| SP0866 | hypothetical protein | X | | C |
| SP0931 | glutamate 5-kinase | | X | C |
| SP0932 | gamma-glutamyl phosphate reductase | X | | L |
| SP0979 | oligoendopeptidase F | X | | H |
| SP1023 | acetyltransferase, GNAT family | X | | H |
| SP1033 | iron-compound ABC transporter, permease protein | X | | L |
| SP1041 | hypothetical protein | X | | C |
| SP1045 | conserved hypothetical protein TIGR00147 | X | | H |
| SP1068 | phosphoenolpyruvate carboxylase | X | X | L |
| SP1111 | conserved hypothetical protein | X | | H |
| SP1121 | 1,4-alpha-glucan branching enzyme | | X | H |
| SP1219 | DNA gyrase subunit A | X | | C |
| SP1396 | phosphate ABC transporter, ATP-binding protein, putative | X | | H |
| SP1398 | phosphate ABC transporter, permease protein, putative | X | | H |
| SP1399 | phosphate ABC transporter, permease protein, putative | X | | H |
| SP1507 | ATP synthase F1, epsilon subunit | X | X | C |
| SP1523 | Snf2 family protein | X | | C |
| SP1544 | aspartate aminotransferase | X | X | H |
| SP1636 | Rrf2 family protein | X | | H |
| SP1637 | conserved hypothetical protein | X | | P |
| SP1645 | GTP pyrophosphokinase | | X | H |
| SP1693 | neuraminidase A, authentic frameshift | X | | 4,C |
| SP1715 | ABC transporter, ATP-binding protein | X | | L,H |
| SP1717 | ABC transporter, ATP-binding protein | X | | H |
| SP1722 | PTS system IIABC components | X | | 5 |
| SP1753 | sodium/dicarboxylate symporter family protein | X | | 6 |
| SP1779 | hypothetical protein | X | | H |
| SP1797 | ABC transporter, permease protein | X | | 5 |
| SP1798 | ABC transporter, permease protein | X | | 5 |
| SP1799 | sugar-binding transcriptional regulator, Lacl family | | X | 5 |
| SP1800 | transcriptional activator, putative | X | | H |
| SP1830 | phosphate transport system regulatory protein PhoU | X | | H |
| SP1856 | transcriptional regulator, MerR family | X | | H |
| SP1870 | iron-compound ABC transporter, permease protein | X | | 7 |
| SP1898 | alpha-galactosidase | X | | H |
| SP1923 | pneumolysin | X | | H |
| SP1952 | hypothetical protein | X | | H |
| SP1964 | DNA-entry nuclease | X | | H |
| SP1972 | membrane protein | X | | P |
| SP2017 | membrane protein | X | | H |
| SP2019 | ABC transporter, ATP-binding protein, truncation | | X | 11 |
| SP2027 | conserved hypothetical protein | X | | 8 |
| SP2052 | competence protein CglB | X | | H |
| SP2054 | conserved hypothetical protein | X | | C |
| SP2086 | phosphate ABC transporter, permease protein | X | | H |
| SP2091 | glycerol-3-phosphate dehydrogenase (NAD(P)+) | X | | L |
| SP2098 | membrane protein | X | X | H |
| SP2116 | conserved domain protein | X | X | P |
| SP2146 | conserved hypothetical protein | X | | P,H |
| SP2150 | ornithine carbamoyltransferase | X | | C |
| SP2163 | PTS system, IIB component | X | | 6 |
| SP2164 | PTS system, IIA component | X | | H |
| SP2169 | zinc ABC transporter, zinc-binding adhesion liprotein | X | | 9 |
| SP2182 | hypothetical protein | X | | H |
| SP2193 | DNA-binding response regulator | X | | 1 |
| SP2231 | ABC transporter, permease protein, putative | | X | H |
| SP2240 | spspoJ protein | X | | 8 |

The proteins encoded by the genes listed in Table 1A-B and 2A-B may be used to produce vaccines or antibodies of the invention. A suitable source of such proteins is for instance *Streptococcus pneumoniae.* The protein may be used non-purified (associated with in intact cells), partially purified (associated with membrane fragments or other cellular constituents), or purified (i.e. isolated and essentially free of other cellular constituents). Having prepared purified or partially purified one or more of the proteins it is possible to prepare a substantially pure preparation of such a protein. Although numerous methods and strategies for protein purification are known in the art it will be most convenient to purify such a protein by either electrophoresis using for instance a sodium dodecylsulphate-polyacrylamide gel (SDS-PAGE) or by affinity chromatography. Each of these methods will be described below.

A protein encoded by a gene listed in Table 1A-B and/or 2A-B may be separated from other proteins by electrophoresis using for instance Tricine-SDS-PAGE (Schagger and Von Jagow (1987) Analytical Biochemistry 166, 368-379) or Glycine-SDS-PAGE (Laemmli (1970) Nature 227, 680-685). Other electrophoresis systems that are capable of resolving the various proteins comprised in a bacterial lysate, or transcribed from its genome and expressed in a suitable expression system, may of course also be employed, such as non-denaturing gel electrophoresis. The area of the PAGE gel including the target protein may be excised and the target polypeptides may be eluted therefrom. The protein of interest may be identified by its mobility relative to reference polypeptides in a gel. To increase purity the eluted protein may be run on a second SDS-PAGE gel and eluted a second time. The protein or peptide contained in the excised gel fragment may then be eluted again and is suitable for use in immunization or in protein sequencing.

The protein may also be purified by affinity chromatography using an antibody (such as a monoclonal antibody) that specifically binds to said protein. The antibody may be covalently coupled to solid supports such as celluloses, polystyrene, polyacrylamide, cross-linked dextran, beaded agarose or controlled pore glass using bifunctional coupling agents that react with functional groups on the support and functional groups (*i*.*e.*, reactive amino acid side chains) on the antibody molecule. Such methods are readily available to the skilled person. The resulting antibody-bearing solid phase is contacted with purified or partially purified protein under reducing conditions using pH, ionic strength, temperature and residence times that permit the protein to bind to the immobilized antibody. The protein is eluted from the column by passing an eluent that dissociates hydrogen bonds through the bed. Buffers at specific pH or NaCl solutions above about 2 M are commonly used eluents.

Methods for carrying out affinity chromatography using antibodies as well as other methods for immunoaffinity purification of proteins are well known in the art (see *e*.*g*., Harlow and Lane, (1988) Antibodies: A Laboratory Manual. Cold Spring Harbor Laboratory, Cold Spring Harbor, NY).

With the teachings provided herein, the skilled person is capable of isolating a protein encoded by a gene listed in Table 1 and/or 2 and test it for its immunogenic properties, e.g. by performing an opsonophagocytosis assay as described in WO 01/12219.

### Antibody production

Antibodies, either monoclonal or polyclonal, can be generated to a purified or partially purified protein or peptide fragment encoded by a gene listed in Table 1 and/or 2 in a variety of ways known to those skilled in the art including injection of the protein as an antigen in animals, by hybridoma fusion, and by recombinant methods involving bacteria or phage systems (see Harlow and Lane (1988) *supra*.; Marks et al., (1992) Journal of Biological Chemistry, 267, 16007-16010; Marks et al., (1992) Biotechnology 10: 779:783; Lowman et al., (1991) Biochem. 30(45): 10832-8; Lerner et al., (1992) Science 258:1313-1314, each of which references discloses suitable methods).

Antibodies against a protein encoded by a gene listed in Table 1 and/or Table 2 or functional homologues thereof, may be produced by immunizing an appropriate vertebrate, preferably mammalian host, *e*.*g*., rabbits, goats, rats and mice or chicken with the protein alone or in conjunction with an adjuvant. Usually two or more immunizations will be involved, and the blood or spleen will be harvested a few days after the last injection. For polyclonal antisera, the immunoglobulins may be precipitated, isolated and (affinity) purified. For monoclonal antibodies, the splenocytes will normally be fused with an immortalized lymphocyte, *e*.*g*., a myeloid line, under selective conditions for hybridomas. The hybridomas may then be cloned under limiting dilution conditions and their supernatants screened for antibodies having the desired specificity. Techniques for producing (monoclonal) antibodies and methods for their preparation and use in various procedures are well known in the literature (see *e.g.* U.S. Pat. Nos. 4,381,292, 4,451,570, and 4,618,577; Harlow, E. and Lane, D. (1988) Antibodies: A Laboratory Manual. Cold Spring Harbor Laboratory, Cold Spring Harbor, NY.; Ausubel, F.M., Brent, R., Kingston, R.E., Moore, D.D.,Seidman, J.G., Smith, J.A., Struhl, K. eds. (1998) Current protocols in molecular biology. V.B. Chanda, series ed. New York: John Wiley & Sons; Rose, N., DeMacrio, E., Fahey, J., Friedman, H., Penn, Gr. (1997) Manual of Clinical Laboratory Immunology. American Soc. Microbiology Press, Washington, D.C Coligan, J.E., Kruisbeek, A.M., Margulies, D.H., Shevach, E.M. Strober, W. (Eds.) (1997) Current Protocols in Immunology. John Wiley & Sons Inc. Baltimore). Typically, an antibody directed against a protein will have a binding affinity of at least 1x10⁵-1x10⁷ M⁻¹.

A recombinant protein or functional homologues thereof, such as may be obtained by expressing a gene from Table 1 and/or 2 in a suitable expression system, is preferred as the antigen in methods for producing an antibody. However, purified proteins may also be used, as well as protein fragments. Antigens suitable for antibody production include any fragment of a protein that elicits an immune response in a mammal exposed to said protein. Preferred antigens of the invention include those fragments that comprise the antigenic determinants, although any region of the proteins encoded by the genes of Tables 1 and/or 2 may in principle be used.

Methods for cloning genomic sequences such as the genes listed in Table 1 and/or 2, for manipulating the genomic sequences to and from expression vectors, and for expressing the protein encoded by the genomic sequence in a heterologous host are well-known, and these techniques can be used to provide the expression vectors, host cells, and the cloned genomic sequences encoding the protein, functional homologues or fragments thereof, which sequences are to be expressed in a host to produce antibodies for use in methods of the present invention (see for instance Sambrook, J., Russell D.W., Sambrook, J. (2001) Molecular Cloning: a Laboratory Manual. Cold Spring Harbor Laboratory Press, Plainview, N.Y., and Ausubel, *et al*., *supra*).

A variety of expression systems may be used to produce antigens for use in methods of the present invention. For instance, a variety of expression vectors suitable to produce proteins in *Escherichia coli, Lactococcus lactis, Bacillus subtilis*, yeast, insect cells, plant cells and mammalian cells have been described, any of which might be used to produce an antigen suitable to be included in a vaccine or useful to produce an antibody or fragment thereof. Of course *Streptococcus pneumoniae* itself may also be used as an expression vector for this purpose.

One use of antibodies of the invention is to provide active ingredients for a pharmaceutical composition capable of inhibiting virulence or growth of a *Streptococcus pneumoniae* pathogen. Another use of antibodies of the invention is to screen cDNA expression libraries for identifying clones containing cDNA inserts that encode proteins of interest or structurally-related, immuno-cross-reactive proteins. Such screening of cDNA expression libraries is well known in the art (see *e*.*g*. Young R.A., Davis, R.W. (1983) Proc. Natl. Acad. Sci. U.S.A. 80:1194-1198), to which reference is made in this context, as well as other published sources. Another use of these antibodies is for use in affinity chromatography for purification of the protein to which it has been elicited or functional homologues thereof. These antibodies are also useful for assaying for infection with *Streptococcus pneumoniae.*

### 1 Antigen Epitopes

The antigen epitopes of this invention, which alone or together form an antigenic determinant of *Streptococcus pneumoniae*, are molecules that are immunoreactive with monoclonal antibodies and whose binding to an antigen of the bacterial pathogen cell prevents the virulence and/or growth of said cell. Systematic techniques for identifying these epitopes are known in the art, as described in US 4,708,871, which is incorporated herein by reference. Typically, these epitopes are short amino acid sequences. These sequences may be embedded in the sequence of longer peptides or proteins, as long as they are accessible.

The epitopes of the invention may be prepared by standard peptide synthesis techniques, such as solid-phase synthesis. Alternatively, the sequences of the invention may be incorporated into larger peptides or proteins by recombinant methods. This is most easily accomplished by preparing a DNA cassette which encodes the sequence of interest, and ligating the cassette into DNA encoding the protein to be modified at the appropriate site. The sequence DNA may be synthesized by standard synthetic techniques, or may be excised from the phage pIII gene using the appropriate restriction enzymes.

Epitopes identified herein may be prepared by simple solid-phase techniques. The minimum binding sequence may be determined systematically for each epitope by standard methods, for example, employing the method described in US 4,708,871. Briefly, one may synthesize a set of overlapping oligopeptides derived from an antigen bound to a solid phase array of pins, with a unique oligopeptide on each pin. The pins are arranged to match the format of a 96-well microtiter plate, permitting one to assay all pins simultaneously, e.g., for binding to a monoclonal antibody. Using this method, one may readily determine the binding affinity for every possible subset of consecutive amino acids.

### 2 Antibody Formulations and Methods of Administration

The antibodies of this invention are administered at a concentration that is therapeutically effective to prevent or treat infections by *Streptococcus pneumoniae*. To accomplish this goal, the antibodies may be formulated using a variety of acceptable excipients known in the art. Typically, the antibodies are administered by injection, either intravenously or intraperitoneally. Methods to accomplish this administration are known to those of ordinary skill in the art. It may also be possible to obtain compositions which may be topically or orally administered, or which may be capable of transmission across mucous membranes.

Before administration to patients, formulants (components other than the active ingredient in a product that can have many functions, such as carrier and excipients) may be added to the antibodies. A liquid formulation is preferred. For example, these formulants may include oils, polymers, vitamins, carbohydrates, amino acids, salts, buffers, albumin, surfactants, or bulking agents.

Additionally, antibodies can be chemically modified by covalent conjugation to a polymer to increase their circulating half-life, for example. Preferred polymers are polyethylene glycol (PEG) and polyoxyethylated polyols, such as polyoxyethylated sorbitol, polyoxyethylated glucose, polyoxyethylated glycerol (POG). The PEG has a preferred average molecular weight between 1,000 and 40,000, more preferably between 2,000 and 20,000, most preferably between 3,000 and 12,000.

Another drug delivery system for increasing circulatory half-life is the liposome. Methods of preparing liposome delivery systems are discussed in Gabizon et al., Cancer Research (1982) 42:4734; Cafiso, Biochem. Biophys. Acta (1981) 649:129; and Szoka, Ann. Rev. Biophys. Eng. (1980) 9:467. Other drug delivery systems are known in the art and are described in e.g., Poznansky et al., Drug Delivery Systems (R. L. Juliano, Ed., Oxford, N.Y. 1980), pp. 253-315; M. L. Poznansky, Pharm. Revs. (1984) 36:277.

After a liquid pharmaceutical composition is prepared, it is preferably lyophilized to prevent degradation and to preserve sterility. Methods for lyophilizing liquid compositions are known to those of ordinary skill in the art. Just prior to use, the composition may be reconstituted with a sterile diluent (Ringer's solution, distilled water, or sterile saline, for example) which may include additional ingredients. Upon reconstitution, the composition is preferably administered to subjects using those methods that are known to those skilled in the art.

As stated above, the antibodies and compositions of this invention are used to treat human patients to prevent or treat *Streptococcus pneumoniae* infections. The preferred route of administration is parenterally. In parenteral administration, the compositions of this invention will be formulated in a unit dosage injectable form such as a solution, suspension or emulsion, in association with a pharmaceutically acceptable parenteral vehicle. Such vehicles are inherently nontoxic and nontherapeutic. Examples of such vehicles are saline, Ringer's solution, dextrose solution, and Hanks' solution. Nonaqueous vehicles such as fixed oils and ethyl oleate may also be used. A preferred vehicle is 5% dextrose in saline. The vehicle may contain minor amounts of additives such as substances that enhance isotonicity and chemical stability, including buffers and preservatives. However, also administration routes other than parenteral (e.g. oral, intranasal, rectal, see herein below with regard to vaccine formulations of the invention) can be applicable for certain embodiments of the invention.

The dosage and mode of administration will depend on the individual. Generally, the compositions are administered so that antibodies are given at a dose between 1 µg/kg and 20 mg/kg, more preferably between 20 µg/kg and 10 mg/kg, most preferably between 1 and 7 mg/kg. Preferably, it is given as a bolus dose, to increase circulating levels by 10-20 fold and for 4-6 hours after the bolus dose. Continuous infusion may also be used after the bolus dose. If so, the antibodies may be infused at a dose between 5 and 20 µg/kg/minute, more preferably between 7 and 15 µg/kg/minute.

The antibody of the present invention may be used prior to infection as a precaution, or after infection has occurred as a therapeutic treatment. Preferably, the therapeutic use of the antibodies as described herein or fragments thereof include administration prior or during the acute invasive phase of the disease.

### Vaccine Formulations and Methods of Administration

The vaccine antigens of this invention are administered at a concentration that is therapeutically effective to prevent or treat infections by *Streptoccus pneumoniae*. To accomplish this goal, the vaccines may be formulated using a variety of acceptable excipients known in the art. Typically, the vaccines are administered by injection, either intravenously or intraperitoneally. Methods to accomplish this administration are known to those of ordinary skill in the art.

Preferably the vaccine contains at least 50 µg of antigenic mass per dose, and most preferably 80 µg per dose. The antigenic mass being the mass of the antigen protein. Vaccines according to the present invention with an antigenic mass up to 275 µg per dose could even be prepared, and such vaccines may still not elicit local reactions at the injection site. Of course even more micrograms of antigen can be put in a vaccine dose of a vaccine according to the invention, but if the protection obtained with the vaccine is not improved with a higher dose the increase in antigenic load only results in the vaccine being more expensive than necessary. In addition an increasing dose of antigen may eventually lead to unacceptable local reactions at the injection site, which should be avoided.

A vaccine according to the invention may contain a (partially) purified or recombinant protein encoded by a gene listed in Tables 1 and/or 2 or an antigenic part thereof, wherein said recombinant protein is preferably produced by way of expression from a expression vector in suitable host cells, said expression vector containing the gene sequence or an immunogenic part thereof under control of a suitable promoter. Several suitable expression systems are known in the art and may be used in a method to prepare a vaccine according to the invention.

A vaccine according to the invention may further comprise a suitable adjuvant. Many adjuvant systems are known in the art, for example commonly used oil in water adjuvant systems. Any suitable oil may be used, for example a mineral oil known in the art for use in adjuvantia. The oil phase may also contain a suitable mixture of different oils, either mineral or non-mineral. Suitable adjuvantia may also comprise vitamin E, optionally mixed with one or more oils. The water phase of an oil in water adjuvanted vaccine will contain the antigenic material. Suitable formulations will usually comprise from about 25-60% oil phase (40-75% water phase). Examples of suitable formulations may comprise 30% water phase and 70% oil phase or 50% of each. Especially preferred is a non-recombinant lactococcal-based vaccine displaying pneumococcal antigens. The lactococcal-derived bacterial shaped particles are non-living and are designated Gram-positive Enhancer Matrix (GEM) particles (Van Roosmalen, M.L. et al., 2006, Methods 38:144-149). These GEM particles are deprived of surface proteins and the intracellular content is largely degraded (Bosma, T. et al., 2006, Appl. Environ. Microbiol. 72:880-889). The GEM particles can be used as anchoring and delivery vehicle for pneumococcal proteins (see Audouy, S.A.L. et al., 2007, Vaccine 25(13):2497).

The vaccine formulations of the present invention may be used in prophylactic methods of the invention by immunizing a subject by introducing said formulations into said subject subcutaneously, intramuscularly, intranasally, intradermally, intravenously, transdermally, transmucosally, orally, or directly into a lymph node. In another embodiment, the composition may be applied locally, near a local pathogen reservoir against which one would like to vaccinate.

The present invention further provides a method for the manufacture of a vaccine intended for the protection of a subject against pneumococcal infection, wherein said vaccine is combined with a pharmaceutically acceptable diluent, carrier, excipient or adjuvant therefore, such that a formulation is provided which can provide a dose of at least 20 µg protein in a single administration event.

A vaccine (prepared by a method) according to the invention can be used in a method to protect a subject against pneumococcal infection.

To provide adequate protection the vaccine is preferably administered in a two shot vaccination regimen, whereby the first shot (priming vaccination) and second shot (boosting vaccination) are given to the subject with a interval of about 3 weeks. In this way the subject will have obtained full protection against pneumococcal infection. The vaccination is very favourable for young children.

A vaccine according to the invention can comprise more than one antigen capable of eliciting antibodies against *Streptococcus pneumoniae.* These antigens can be chosen from the proteins encoded by the genes listed in Table 1 and/or 2, or aditionally known antigens, such as those listed in the introduction above may be added.

Further, the genes of Table 1 and/or 2 and/or the proteins encoded by said genes provide excellent targets for small chemical molecules. For finding novel antibiotic compounds a screen with any of these genes and proteins would be suitable.

### EXAMPLES

### A. ANIMAL INFECTION MODELS

### 1.1.1. Mouse infection model

Nine-week old, female outbred CD-1 mice (Harlan, Horst, the Netherlands) were used for the mouse infection experiments. In the bacteraemia model, mice were infected in a tail vein with a 100 µl inoculum. At predetermined times after infection (0.5, 12, and 24 hours), groups of mice were sacrificed by injection anaesthesia, and bacteria were recovered from the blood by a retro orbital puncture. The number of viable bacteria in the blood was determined by plating serial dilutions on agar plates. All mouse infection experiments were performed with approval of the Radboud University Nijmegen Medical Centre Committee for Animal Ethics.

### 1.1.2. Rabbit infection model

Outbred New Zealand white rabbits weighing approximately 2,500 g were used for the rabbit meningitis infection model. On the day before the experiment the rabbits were prepared for fixation in stereotactic frames. Rabbits were anaesthetized with dormicum 0.5 ml/kg (midazolam 5 mg/ml) subcutaneously and after 10 minutes with Hypnorm 0.35ml/kg (fentanylcitrat + fluanison) intramuscularly. Ears, scalps and necks were shaved and the skin disinfected. On each rabbit an incision measuring approximately 2 cm was made on the forehead and the scalp was exposed by blunt dissection. 4 bore holes were made demarcating a square and 4 screws were screwed up at right angles to the surface (2.5-3 turns). An acrylic helmet, embedding a turnbuckle, was moulded from dental casting material directly onto the scalp of the rabbit and the helmet was cooled under running water while stiffening. Rabbits were put back into their cages to rest for 16-18 hours until the beginning of the experiment. In the post-operative period buprenorhine was given for pain-treatment as in injection upon conclusion of the operative procedure. On the day of the experiment rabbits were anaesthetized with urethan 3.5 ml/kg (dimethyl-acrylat 50%, 1.75 g/kg) subcutaneously. Veneous catheters were applied in left ear-veins and Mebumal approximately 0.5-1 ml (pentobarbital 50 mg/ml), was infused slowly until the rabbits were asleep and deeply anaesthetized. Three-way taps were connected to the venous catheters and syringes containing pentobarbital for supplemental anaesthetics and isotonic NaCl with heparin 1 UI/ml for flushing were connected to the taps. Rabbits were observed every 1-2 hours. If need of supplemental anaesthesia arose (reaction upon squeezing the tail) additional 0.2 ml Mebumal (pentobarbital 50 mg/ml) was given. Arterial canules were applied in the right ear arteries and were used for blood-sampling throughout the experiment. Bolts were fastened to the turnbuckles embedded in the acrylic helmets and used for fixation of the rabbits in stereotactic frames. Rabbits remained fixated throughout the experiments. Cisterna magna was punctured by a spinal-canula fastened to the stereotactic frame. 0.2 ml of CSF was removed and the bacterial inoculum of bacterial cells suspended in 20 µl beefbroth was injected. The canula was left in place throughout the experiment and used for repeated CSF-sampling. At predetermined times after bacterial inoculation (3, 9, and 15 hours), 0.3 ml of CSF and 1-2 ml of blood was aspirated and transferred to EDTA / Eppendorf-tubes. After 15 hrs, sampling experiments were concluded by euthanizing the rabbits with an overdose of Mebumal (50 mg in 1 ml). All rabbit infection experiments were performed with approval by the Danish Animal Inspectorate (Dyreforsoegstilsynet).

### B. GENOMIC ARRAY FOOTPRINTING

***DNA isolation.*** Chromosomal DNA was isolated from pneumococcal cultures by cetyl-trimethylammonium bromide (CTAB) extraction using standard protocols.

***Generation of transposon mutant libraries.*** For *in vitro* transposon mutagenesis, 1 µg of pneumococcal DNA was incubated in the presence of purified HimarC9 transposase with 0.5 µg of plasmid pR412T7 (Bijlsma, J.J.E. et al., 2007, Appl. Environm. Microbiol. 73(5):1514-1524) as donor for *mariner* transposon conferring spectinomycin resistance. After repair of the resulting transposition products with T4 DNA polymerase and *Escherichia coli* DNA ligase, the DNA was used for transformation of strain TIGR4. Preparation and transformation of precompetent *Streptococcus pneumoniae* cell stocks was performed essentially as described. Briefly, cCAT medium was inoculated with several colonies and grown to an optical density at 620 nm (OD₆₂₀) of 0.25-0.3. After a 30-fold dilution of the culture in CTM medium, cells were grown to an OD₆₂₀ of 0.1, pelleted, resuspended in 0.1 volume of CTM-pH7.8 (CTM adjusted to pH 7.8 with NaOH) containing 15% glycerol, and stored at -80°C. For transformation, precompetent TIGR4 cells were grown for 15 minutes at 37°C in a 10-fold volume of CTM-pH7.8 supplemented with 100 ng/ml CSP-2. After addition of DNA, cultures were incubated for 30 min at 32°C, followed by a two-hour incubation at 37°C. After overnight growth on selective plates containing 150 µg/ml spectinomycin, the required number of colonies formed by the transposon mutants were scraped from the plates, pooled, grown to mid-log phase in 20 ml of GM17 medium supplemented with spectinomycin, and stored at -80°C.

***Probe generation, labeling, and microarray hybridization.***

Chromosomal DNA from challenged and non-challenged mutant libraries was digested with *Alu*I endonuclease. The resulting DNA fragments were purified using Qiagen MinElute columns and used as a template for an *in vitro* T7 RNA polymerase reaction using the Ambion T7 MegaScript kit. After removal of template DNA by DNAseI treatment, RNA was purified using Qiagen RNeasy MinElute columns. Fluorescent Cy3/Cy5-labeled dUTP nucleotides were incorporated by reverse transcription using Superscript III. Labeled challenged cDNA was mixed with labeled non-challenged cDNA and purified by washing and ultrafiltration using GFX and Microcon-30 spin columns. Samples were suspended in Slidehyb buffer 1 and hybridized in to pneumococcal microarrays for 16 hours at 45°C. Microarrays used in this study were constructed as described and contain amplicons representing 2,087 ORFs of *Streptococcus pneumoniae* TIGR4 as well as several 70-mer oligos specific for *Streptococcus pneumoniae* strains D39, R6, G54, 23F, OXC14, INV200, and INV104B, all spotted in duplicate. After hybridization, microarrays were washed with 2xSSC, 0.25% SDS for 5 min, followed by 2 washes in 1x SSC and 0.5x SSC for 5 min each. Finally, slides were dipped into H₂O and dried by centrifugation for 5 min at 50 x g.

***Microarray data analysis.*** Dual channel array images were acquired on a GenePix 4200AL microarray scanner and analyzed with GenePix Pro software. Spots were screened visually to identify those of low quality and removed from the data set prior to analysis. A net mean intensity filter based on hybridization signals obtained with oligomer-spots representing open reading frames unique for *Streptococcus pneumoniae* strain R6 was applied in all experiments. Slide data were processed and normalized using MicroPreP. Further analysis was performed using a Cyber-T implementation of the Student's *t* test. This web-based program lists the ratios of all intra-replicates (duplicate spots) and inter-replicates (different slides), the mean ratios per gene, and standard deviations and (Bayesian) p-values assigned to the mean ratios. For identification of conditionally essential genes in the bactaeremia model, only genes with a minimum of 6 reliable measurements and a Bayesian *p*-value < 0.001 were included. Furthermore, an average fold-change cut-off of 3.0 in a minimum of two time-points, or 5.0 in one time-point was applied. For identification of conditionally essential genes in the meningitis model, genes were included with a Bayesian p-value < 0.001 and a minimum of 5 or 6 reliable measurements when, respectively, 6 or 8 measurements were available. Furthermore, an average fold-change cut-off of 2.5 in a minimum of two time-points, or 4.0 in one time-point was applied.

***In silico analyses.*** Annotation of genes was derived from the TIGR Comprehensive Microbial Resource database (http://cmr.tigr.org/tigr-scripts/CMR/CmrHomePage.cgi). The computational prediction of subcellular localization of proteins encoded by genes identified in GAF screens was performed using several prediction servers, such as SignalP3.0 (http://www.cbs.dtu.dk/services/SignaIP), PSORTb (http://www.psort.org), and TMHMM (http://www.cbs.dtu.dk/services/TMHMM).

### C. EXPERIMENTAL DESIGN

### 1.3.1. Genes essential for survival in the blood stream

To identify genes essential for the pneumococcus *in vivo* specifically for survival in blood, four independent *Streptococcus pneumoniae* TIGR4 *mariner* transposon mutant libraries consisting of 1,000-2,000 independent transposon mutant colonies were used to infect groups of twelve CD-1 mice in a murine bacteraemia model of infection, e.g., a 100 µl-inoculum containing 1x10⁶ colony forming units (CFU) administered intravenously. At three time-points post-infection, namely 0.5, 12, and 24 hours, four mice from each group were sacrificed and blood was collected. Bacterial load in each sample was determined by plating serial dilutions, and the remainder was stored in 15% glycerol at -80°C. Before DNA isolation and GAF, samples were grown *in vitro* to mid-log phase in GM17 medium supplemented with spectinomycin. GAF analysis of the blood samples resulted in identification of several mutants that had disappeared from blood during challenge at one or more of the time-points sampled. The corresponding genes can be considered potential novel targets identified by *in vivo* GAF, i.e., *Streptococcus pneumonia* genes essential for survival in the blood. These genes are listed in Tables 1A-B.

### 1.3.2. Genes essential for survival in cerebrospinal fluid

To identify genes essential for the pneumococcus *in vivo* specifically for survival in CSF, the same four independent *Streptococcus pneumoniae* TIGR4 *mariner* transposon mutant libraries consisting of 1,000-2,000 independent transposon mutant colonies that were used for the bacteraemia model were used to infect groups of four Outbred New Zealand white rabbits in a rabbit meningitis model of infection, e.g., a 20 µl-inoculum containing 1x10⁶ CFU administered into the cisterna magna. At 3, 9, and 15 hours after bacterial inoculation, 0.3 ml of CSF was collected from each rabbit. Bacterial load in each CSF sample was determined by plating serial dilutions, and the remainder was stored in 15% glycerol at -80°C. Before DNA isolation and GAF, CSF samples were grown *in vitro* to mid-log phase in GM17 medium supplemented with spectinomycin. GAF analysis of the CSF samples resulted in identification of several mutants that had disappeared from CSF during challenge at one or more of the time-points sampled. The corresponding genes can be considered potential novel targets identified by *in vivo* GAF, i.e., *Streptococcus pneumoniae* genes essential for survival in the CSF. These genes are listed in Tables 2A-B.

## Claims

1. A vaccine formulation providing protection against pneumococcal infection in a subject, said formulation comprising an effective amount of a protein encoded by a gene listed in Table 1 (1A and 1B) and/or Table 2 (2A and 2B) or a functional homologue or an immunogenic part thereof together with at least one of a pharmaceutically acceptable diluent, carrier, excipient or adjuvant therefore.

2. A formulation according to claim 1, wherein said immunogenic part is an antigenic determinant of said pathogen.

3. A formulation according to claim 1 or 2, wherein said protein is encoded by a gene listed in Table 1A, Table 1B, Table 2A, and/or Table 2B.

4. A formulation according to claim 3, wherein said protein is encoded by a gene listed in two or more of Table 1A or Table 1B and Table 2A or Table 2B

5. A formulation according to any one of claims 1-4, wherein said formulation provides protections against pneumonia, meningitis, otitis media and/or sepsis caused by *Streptococcus pneumoniae.*

6. A protein encoded by a gene listed in Table 1 and/or 2 or an immunogenic part thereof, for use as a vaccine.

7. An antibody against a protein encoded by a gene listed in Table 1 and/or 2 or fragment thereof, preferably a humanized antibody or fragment thereof.

8. An antibody or fragment thereof, preferably a humanized antibody or fragment thereof according to claim 7, for use as a medicament for the prophylactic or therapeutic treatment of a pneumococcal infection in a subject.

9. Use of an antibody or fragment thereof, preferably a humanized antibody or fragment thereof according to claim 7, for the manufacture of a medicament for the prophylactic or therapeutic treatment of a pneumococcal infection in a subject.

10. A pharmaceutical composition comprising an antibody or fragment thereof, preferably a humanized antibody or fragment thereof according to claim 7, and a pharmaceutically acceptable carrier.

11. A method for prophylactic or therapeutic treatment of a pneumococcal infection in a subject comprising administering to a subject in need of such treatment an effective amount of a vaccine formulation as defined in any one of claims 1-5 and/or an effective amount of a pharmaceutical composition as defined in claim 10.

12. A method for preparing an pneumococcal vaccine formulation, the said method comprising bringing into association, an effective amount of a protein encoded by a gene listed in Table 1 and/or Table 2 or an immunogenic part thereof and at least one of a pharmaceutically acceptable diluent, carrier, excipient or adjuvant therefore.

13. A method for preparing a pharmaceutical composition as defined in claim 10, the said method comprising bringing into association, an effective amount of an antibody, preferably a humanized antibody, or fragment thereof, according to claim 7 and a pharmaceutically acceptable carrier.
